# EUROPEAN PATENT SPECIFICATION

(11) **EP 2 203 104 B1**
(45) Date of publication and mention of the grant of the patent: **16.11.2016**
(21) Application number: 08837340.2
(22) Date of filing: 08.10.2008
(51) Int. Cl.: A61B 1/00, A61B 17/34

(54) **SYSTEMS, DEVICES AND METHODS HAVING AN OVERTUBE FOR ACCESSING A BODILY OPENING**
SYSTEME, VORRICHTUNGEN UND VERFAHREN MIT ÜBERTUBUS FÜR DEN ZUGANG ZU EINER KÖRPERÖFFNUNG
SYSTÈMES, DISPOSITIFS ET PROCÉDÉS PRÉSENTANT UN SURTUBE POUR ACCÉDER À UNE OUVERTURE CORPORELLE

(30) Priority: 09.10.2007 US 978751 P
(43) Date of publication of application: 07.07.2010
(73) Proprietor: Cook Medical Technologies LLC, Bloomington, IN 47404 (US)
(72) Inventor: SURTI, Vihar, C., Winston-salem NC 24104 (US)
(74) Representative: Wächter, Jochen
(86) International application number: PCT/US2008/079199
(87) International publication number: WO 2009/048948

(56) References cited:
- WO-A-2004/000410
- WO-A-2004/037097
- US-A- 4 023 559
- US-A- 5 354 302
- US-A- 5 814 073
- US-A1- 2005 149 078

## Description

### FIELD OF THE INVENTION

The present invention relates generally to medical systems utilizing an overtube to provide access to a bodily opening through a bodily lumen, such as for an endoscope and other medical devices.

### BACKGROUND OF THE INVENTION

Openings in bodily walls may be formed to gain access to adjacent structures of the body, such techniques being commonly referred to as translumenal procedures. For example, culdoscopy was developed over 70 years ago, and involves transvaginally accessing the peritoneal cavity by forming an opening in the *cul de sac.* This access to the peritoneal cavity allows medical professionals to visually inspect numerous anatomical structures, as well as perform various procedures such as biopsies or other operations, such as tubal ligation. Many transluminal procedures for gaining access to various body cavities using other bodily lumens have also been developed. For example, the bodily lumen(s) of the gastrointestinal tract are often endoscopically explored and can be utilized to provide access to the peritoneal cavity and other body cavities, all in a minimally invasive manner. US 2008/020 8224 A1 discloses such a procedure.

Although transluminal procedures are minimally invasive, there are also various risks involved. For example, when an opening is formed in a bodily wall of the gastrointestinal tract, such as in the stomach or intestines, spillage of the stomach contents, intestinal contents or other bodily fluids into the adjacent body cavity can occur. Travel of bacteria laden fluids outside of the gastrointestinal tract may cause unwanted and sometimes deadly infection.

Prior art medical systems for accessing bodily openings have been developed and include a medical instrument and an overtube, as described in US-A-5 354 302, WO 2004/000410 A, US-A-5 814 073 and US-A-4 023 559. The known overtubes are sized to receive the medical instrument and comprise a distal end having a convexly or conically shaped portion and a plurality of longitudinally slits defining a plurality of flaps. By distally translating the medical instrument through the distal end, the flaps can be moved radially between an introduction configuration and an operative configuration. The known medical systems are used for accessing a tissue area within the tissue or for expanding and keeping the bodily opening in the expanded state.

US 2005/149078 A1 discloses an overtube having a distal end with a pointy tip for cutting through the tissue to form a bodily opening and a radially extended portion for cutting away tissue surrounding the bodily opening when retracting the overtube.

### BRIEF SUMMARY OF THE INVENTION

It is the general object of the present invention to provide medical systems for accessing a bodily opening that are safe, reliable and repeatable.

This object is solved by the subject matter of claim 1.

A medical system for accessing a bodily opening is provided in accordance with the teachings of the present invention, and generally includes a medical instrument and an overtube. The overtube defines an overtube lumen that is sized to receive the medical instrument. The overtube includes a distal end having a tapered portion and a plurality of longitudinally extending slits defining a plurality of flaps. The tapered portion of the overtube is sized relative to the medical instrument such that distal translation of the medical instrument through the distal end of the overtube forces the plurality of flaps to move radially outwardly.

According to more detailed aspects, the distal end of the overtube forms a fluidic seal with the tissue. The plurality of flaps may form the fluidic seal with the tissue. Each of the plurality of flaps includes side edges, and the side edges of adjacent flaps abut each other. When the plurality of flaps move radially outwardly, the tissue extends between the flaps and forms a fluidic seal with the endoscope. A portion of the overtube that is proximal to the plurality of flaps may also form the fluidic seal with the tissue. The longitudinal length of the flaps is preferably less than or about equal to a thickness of the tissue defining the bodily opening.

The overtube generally comprises a tubular body defining an overtube lumen and a longitudinal axis. The tubular body has a tapered distal end, which in turn has a plurality of longitudinally extending slits defining a plurality of flaps. The overtube is operable between an introduction configuration and an operative configuration. The plurality of flaps move radially outwardly from the introduction configuration to the operative configuration. A distal tip of the distal end of the overtube flares radially outwardly. The flared distal tip is configured to engage the bodily opening, and more particularly to frictionally engage a distal side of a bodily wall in the proximity of the bodily opening.

During operation, the overtube is translated through the bodily lumen. The overtube is moved through the bodily opening, and the medical instrument is translated through the overtube such that the plurality of flaps move radially outwardly.

According to more detailed aspects a surgical tool may be translated through the distal end of the overtube and used to form the bodily opening. The medical instrument is preferably an endoscope, and the surgical tool may be translated through a working channel of the endoscope and through the distal end of the overtube while the endoscope is positioned within the overtube. After forming the bodily opening, the surgical tool is retracted, and the overtube is translated through the bodily opening. The endoscope may then be translated through the overtube. The step of forming the bodily opening may include manipulating the overtube and the surgical tool together such that the distal end of the overtube serves to shield a portion of the surgical tool.

### BRIEF DESCRIPTION OF THE DRAWINGS

FIG. 1 is a cross-sectional view of a medical system constructed in accordance with the teachings of the present invention;
FIG. 2 is a side view of an overtube forming a portion of the medical system depicted in FIG. 1;
FIG. 3 is a cross-sectional view showing use of the medical system depicted in FIG. 1;
FIG. 4 is another cross-sectional view showing use of the medical system depicted in FIG. 1;
FIG. 5 is a cross-sectional view taken about the line 5-5 in FIG, 4;
FIG. 6 is yet another cross-sectional view showing use of the medical system depicted in FIG. 1;
FIG. 7 is a cross-sectional view taken about the line 7-7 in FIG. 6;
FIG. 8 is a cross-sectional view of an alternate embodiment of the medical system depicted in FIG. 1, constructed in accordance with the teachings of the present invention.

### DETAILED DESCRIPTION OF THE INVENTION

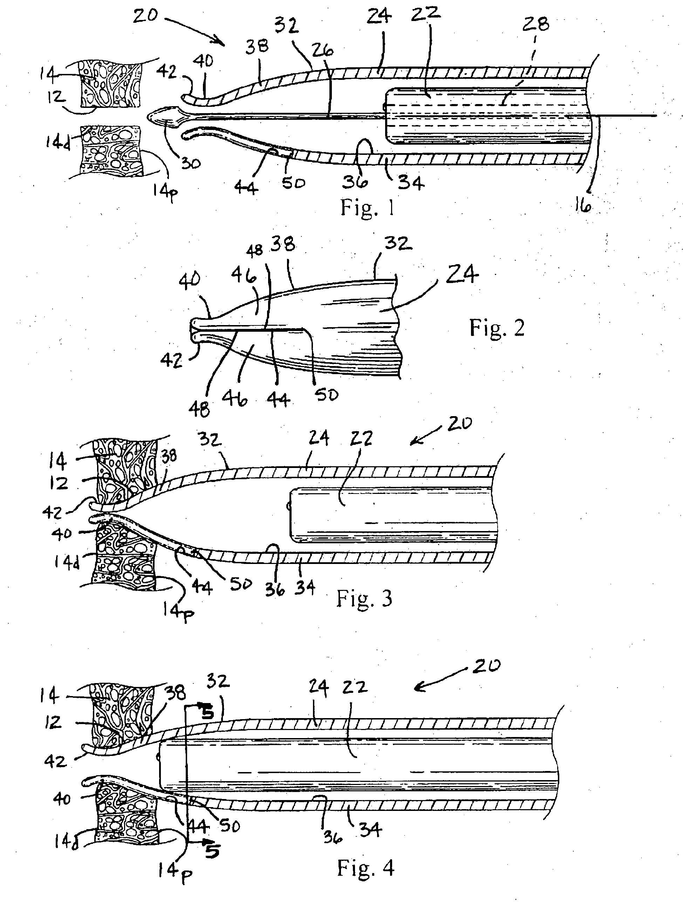

Turning now to the figures, FIG. 1 depicts a cross-sectional view of a medical system 20 for accessing a bodily opening 12 defined by tissue 14, constructed in accordance with the teachings of the present invention. The medical system 20 generally includes an endoscope 22 and an overtube 24 for accessing the opening 12. While the medical system 20 has been depicted as including the endoscope 22, many different medical instruments may be used in conjunction with the overtube 24, such as wire guides, catheters, needles, device deployment systems, biopsy devices and the like. For example, in FIG. 1 the opening 12 in the tissue 14 has been depicted as formed utilizing a surgical tool 26, which can be employed in conjunction with the overtube 24, and with or without the endoscope 22.

The surgical tool 26 is preferably an electrosurgical cutting tool that has been traversed through a working channel 28 of the endoscope 22, although it will be recognized by those skilled in the art that any type of cutting device may be employed to form the opening 12. The surgical tool 26 includes a cutting tip 30 which projects from a distal end 32 of the overtube 24 for forming the opening 12, as will be discussed in greater detail herein. While the opening 12 has been described as an intentionally formed perforation, it will be recognized by those skilled in the art that the bodily opening 12 may be unintentionally formed or naturally occurring. Alternatively, bodily opening 12 may be an opening that is part of the gastrointestinal tract or other bodily lumen such as the openings at the esophageal sphincter, the pylorus sphincter, the sphincter of oddii, the ileocecal valve, or the anus.

With reference to both FIGS. 1 and 2, the overtube 24 includes a tubular body 34 defining an overtube lumpen 36 that is sized to receive the endoscope 22 for translation therein. The overtube 24 and overtube lumen 36 define a longitudinal axis 16 along which the endoscope 22 is received. The overtube 24 may be constructed of a variety of plastic materials, such as polytetrafluorethylene (PTFE), polyethylene ether ketone (PEEK), polyamide, nylon, polyimide, polyurethane, including multi-layer or single layer constructions with or without reinforcement wires, coils or filaments. The overtube lumen 36 is preferably sized about the same size or larger than the endoscope 22 to permit relative translation therebetween. Depending on the size and construction of the endoscope 22, the inner diameter of the overtube 24 may be 5% to 40% larger than the outer diameter of the endoscope 22. The overtube 24 has a length suitable for accessing the particular anatomy desired, but is generally shorter than the working length of the endoscope 22. The proximal end of the overtube 24 may include a gripping surface or otherwise be attachable to the endoscope 22.

The distal end 32 of the overtube 24 is tapered and narrows as it approaches the distal tip 40 of the overtube 24. Likewise, the overtube lumen 36 has a diameter which decreases along its length at the distal end 32, as does the outer diameter of the tapered portion 38. The distal end 32 has been shown as having a compound curvature, namely the tapered portion 38 is generally concave while the distal tip 40 is generally convex, although it will be recognized that the distal end 32 may simply have a convex curvature, a concave curvature, a complex curvature or different compound curvature, or may have no curvature, such as by following a generally straight line to form a conical or pyramidal shape. The distal tip 40 of the overtube 24 is flared radially outwardly to define a lip 42. The lip 42 is structured for engagement of the tissue 14 around the bodily opening 12, as will be discussed further herein.

A plurality of slits 44 are also formed in the distal end 32 of the overtube 24, and extend along the tapered portion 38 of the distal end 32. The plurality of slits 44 extend longitudinally to define a plurality of flaps 46 between the slits 44. The plurality of slits 44 and plurality of flaps 46 allow the overtube 24 to be operated between an introduction configuration and an operative configuration. The introduction configuration is shown in FIGS. 2 and 3, while various states of the operative configuration are shown in FIGS. 1 and 4 through 7. In the introduction configuration, the flaps 46 engage each other at distal tip 40 of the overtube 24 to substantially close the overtube lumen 36. Generally, the plurality of flaps 46 move radially outwardly (away from the longitudinal axis 16) as various medical instruments or devices such as the surgical tool 26 or endoscope 22 are translated through the distal end 32 of the overtube 24, thus opening the overtube lumen 36. For example, the reduced diameter of the overtube lumen 36 in the area of the distal end 32 is smaller than an outer diameter of the endoscope 22, such that passage of the endoscope 22 through the distal end 32 causes the endoscope 22 to press against the plurality of flaps 46 and move them radially outwardly into the operative configuration. Due to the taper of the distal end 32, many differently sized medical instruments and devices may be used in conjunction with the overtube 24.

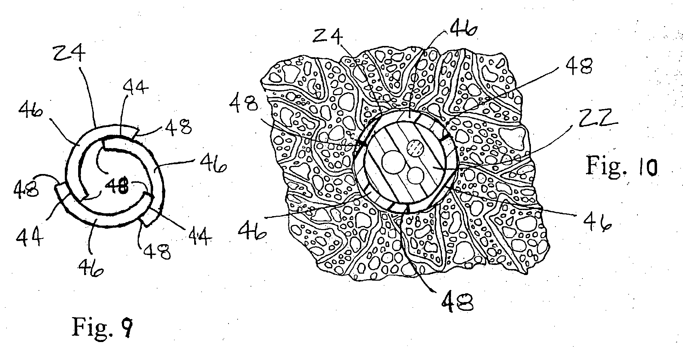

As will be described in more detail below, the medical system 20 and overtube 24 are particularly adapted for gaining access to the bodily opening 12 and forming a fluidic seal with the tissue 14 to restrict the flow of bodily fluids through the opening 12. The endoscope 22 or other medical instrument may be repeatedly passed through the distal end 32 of the overtube 24 and the opening 12 in the tissue 14 while substantially maintaining the fluidic seal between the tissue 14 and the medical system 20. The plurality of flaps 46 include side edges 48. The side edges 48 of adjacent flaps 46 abut each other in the introduction configuration shown in FIGS. 2 and 3, and thus are substantially sealed against one another. However, it will also be recognized that the flaps 46 may be sized such that the side edges 38 abut each other in the operative configuration, as shown in FIGS. 9 and 10. For example, the flaps 46 are sized such that their side edges 38 overlap in the introduction configuration shown In FIG. 9, while the side edges abut each other in the operative configuration shown in FIG. 10.

The usage of the medical system for accessing the bodily opening 12 via a bodily lumen will now be described with reference to FIGS. 1-8. By way of example, the bodily lumen could be all or a portion of the gastrointestinal tract, such as the mouth, esophagus and stomach. This lumen may be used to access the tissue 14 of the gastric wall, although the method may be performed with many different bodily lumens and openings. The endoscope 22 and overtube 24 are provided, and together the endoscope 22 and the overtube 24 are translated through the bodily lumen to a position proximate the tissue 14, as shown in FIG. 1. In a preferred construction, the overtube 24 is formed of a transparent or translucent plastic material such as those described above, allowing the endoscope 22 to visualize the bodily lumen and target site from within the overtube 24. Likewise, the endoscope 22 or other medical tool may be advanced beyond distal tip 40 of the overtube 24 anytime direct visualization is desired, such as during introduction or for forming and accessing the opening 12. Of course, the overtube 24 may also be introduced alone, and would preferably includes positional markers, such as those visible under fluoroscopy, ultrasound, or other remote visualization techniques known in the art.

In situations where it is desired to form the opening 12, the surgical cutting tool 26 may be translated through the working channel 28 of the endoscope 22 and through the distal end 32 of the overtube 24. Preferably, the surgical tool 26 is positioned such that the cutting tip 30 projects beyond the distal tip 40 of the overtube 24, while the remainder of the surgical tool 26 is positioned within the overtube 24. As such, the overtube 24, and particularly the plurality of flaps 46 at the distal end 32, serve to shield a portion of the surgical tool 26. The surgical tool 26 may be manipulated to form the opening 12, and preferably the overtube 24 and surgical tool 26 are moved together and in unison such that the overtube 24 continues to shield the surgical tool 26 during the cutting step. The surgical tool 26 is then retracted through the working channel 28 of the endoscope 22. It will be recognized that if the surgical tool 26 is of the type that is back-loaded into endoscope 22, both the endoscope 22 and surgical tool 26 may be withdrawn together from the overtube 24 and the surgical tool 26 removed from the endoscope 22. The endoscope 22 would then be reinserted through the overtube 24.

As best seen in FIG. 3, the overtube 24 is moved through the bodily opening 12 and the tissue 14. In particular, the flared distal tip 40 is moved beyond a distal side 14d of the tissue 14 and of the opening 12. The tissue 14 exhibits a natural elasticity that causes the tissue 14 to be biased radially inwardly towards the opening 12. That is, the tissue 14 will naturally try to close the opening 12. Accordingly, the tissue 14 naturally presses against the distal end 32 of the overtube 24, and in particular the plurality of flaps 46. As such, a fluidic seal is formed between the overtube 24 and tissue 14. As illustrated, the plurality of slits 44 have a proximal end 50 which is positioned on the proximal side 14p of the tissue 14 and of the opening 12. At the same time, the side edges 48 of the plurality of flaps 46 abut each other to seal and substantially prevent the passage of bodily fluids therethrough. The lip 42 of the flared distal tip 40 frictionally engages the distal side 14d of the tissue 14 to resist the withdrawal (i.e. distal translation) of the overtube 24 through the opening 12.

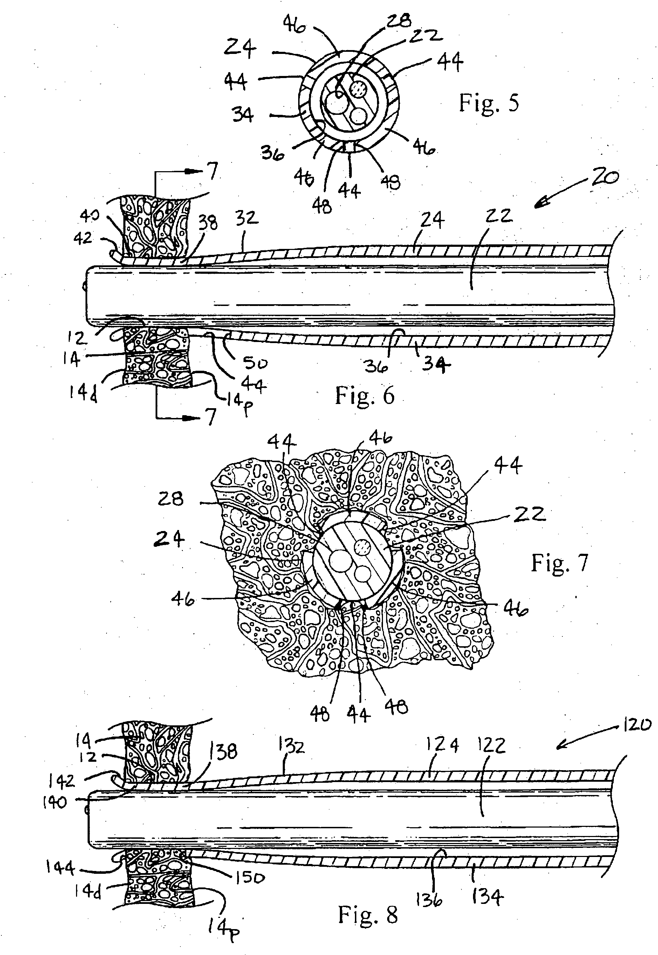

As best seen in FIG. 4, the endoscope 22 is then translated through the overtube 24 such that the plurality of flaps 46 begin to move radially outwardly. That is, the distal end of the endoscope 22 presses against the plurality of flaps 46 and forces them radially outwardly against the pressure of the tissue 14. In this stage of the operative configuration of the overtube 24, a space 50 between the plurality of flaps 46 forms as the plurality of flaps 46 move radially outwardly, as best seen in the cross-sectional view of FIG. 5.

As seen in FIGS. 6 and 7, the endoscope 22 is further advanced through the distal end 32 of the overtube 24, such that the endoscope 22 is moved through the opening 12 and positioned distally of the distal side 14d of the tissue 14. In this stage of the operative configuration, the plurality of flaps 46 are again pressed against the tissue 14 within the opening 12, while the tissue 14 resists the outward movement of the flaps 46. Due to this natural elasticity of the tissue 14, a fluidic seal is formed between the tissue 14 and the overtube 24 as well as between the tissue 14 and the endoscope 22. That is, the tissue 14 extends into the spaces 50 between the plurality of flaps 46 and directly presses against the endoscope 22 to form a fluidic seal therebetween.

During application, the lip 42 defined by the flared distal tip 40 remains frictionally engaged with the distal side 14d of the tissue 14 and opening 12. This maintains the position of the overtube 24 within the opening 12. In this operative configuration, the endoscope 22 may be manipulated to visually inspect a target area and/or used in conjunction with additional surgical instruments (i.e. such as through its working channel 28) and various operations or procedures performed. The endoscope 22 may be withdrawn through the overtube 24 and away from the distal end 32 such that the overtube 24 and its plurality of flaps 46 return to the introduction configuration depicted in FIGS. 2 and 3. The endoscope 22 or other A medical instruments may be inserted, withdrawn and reinserted through the overtube 24 to access the opening 12 as many times as needed or desired. At the same time, the overtube 24 maintains its access to the opening 12 while providing a fluidic seal with the tissue 14.

Whenever access to the opening 12 is no longer needed, the overtube 24 may be translated proximally by overcoming the friction between flared distal tip 40 and the tissue 14. An appropriate suturing tool may be utilized to close the opening 12 if needed. Exemplary suturing devices and perforation closure methods are disclosed in US 2008/0132948 A1, US 2009/0048613 A1, US 2009/0069847 A1 and US 2008/0300629 A1.

Another embodiment of a medical system 120, constructed in accordance with the teachings of the present invention, has been depicted in FIG. 8. In this embodiment the endoscope 122 and overtube 124 are of substantially similar construction as the prior embodiment, except for the distal end 132 of the overtube 124, which has a different construction. In particular, the plurality of slits 144 have proximal ends 150 that are ideally located within the opening 12 in the tissue 14. That is, the tissue 14 presses against a portion of the overtube 24 that is located proximal to the plurality of slits 144 (i.e. proximal to their proximal ends 150). Preferably, the longitudinal length of the slits 144, and hence the length of the plurality of flaps 146, are sized to be about equal to or less than the thickness of the tissue 14. As such, the flared distal tip 140 and its lip 142 still serve to engage the distal side 14d of the tissue 14 and opening 12. It will be recognized that the fluidic seal between the overtube 124 and tissue 14, which substantially blocks the passage of contents through the opening 12, is present without regard to whether the plurality of flaps 146 are in the introduction configuration or the operative configuration. Although the tissue 14 will still extend between the plurality of flaps 146 and directly engage the endoscope 122, a fluidic seal is formed between the overtube 124 and tissue 14 irrespective of the presence or position of the endoscope 122.

Accordingly, it will be seen that the medical systems of the present invention provide access to a bodily opening in a manner that is safe, reliable and easily repeatable. An endoscope or various other medical instruments may be repeatedly passed through an overtube to access the opening and structures on a distal side of the opening as needed. Further, the overtube is easily deployable, provides an effective fluidic seal with the tissue defining the opening, and is easily removed.

The foregoing description of various embodiments of the invention has been presented for purposes of illustration and description. It is not intended to be exhaustive or to limit the invention to the precise embodiments disclosed. Numerous modifications or variations are possible in light of the above teachings. The embodiments discussed were chosen and described to provide the best illustration of the principles of the invention and its practical application to thereby enable one of ordinary skill in the art to utilize the invention in various embodiments and with various modifications as are suited to the particular use contemplated. The use of the terms seal or fluidic seal do not require that the barrier is completely leakproof, but that it substantially prevents the flow of fluid or other contents therethrough. All such modifications and variations are within the scope of the invention as determined by the appended claims.

## Claims

1. A medical system (20, 120) for accessing a bodily opening (12) defined by tissue (14), the medical system (20, 120) comprising:
a medical instrument (22, 122); and
an overtube (24, 124) defining an overtube lumen (36) sized to receive the medical instrument (22, 122), the overtube (24, 124) and the overtube lumen (36) defining a longitudinal axis (16), the overtube (24, 124) including a distal end (32, 132) having a tapered portion (38) and a plurality of longitudinally extending slits (44, 144) defining a plurality of flaps (46, 146), the overtube (24, 124) having a diameter that is reduced along the tapered portion (38) of the distal end (32, 132),
the tapered portion (38) of the overtube (24, 124) sized relative to the medical instrument (22, 122) such that distal translation of the medical instrument (22, 122) through the distal end (32, 132) of the overtube (24, 124) forces the plurality of flaps (46, 146) to move radially between an introduction configuration and an operative configuration,
the plurality of flaps (46, 146) engaging each other at a distal tip (40, 140) of the overtube (24, 124) to substantially close the overtube lumen (36) in the introduction configuration, the plurality of flaps (46, 146) moved radially outwardly in the operative configuration,
**characterized in that**
the distal tip (40, 140) is flared radially outwardly along a continuous curvature to define a lip (42, 142).

2. The medical system (20, 120) of claim 1, wherein the distal end (32, 132) of the overtube (24, 124) forms a fluidic seal with the tissue (14).

3. The medical system (20, 120) of claim 2, wherein each of the plurality of flaps (46, 146) include side edges (48), and wherein the side edges (48) of adjacent flaps (46, 146) abut each other in the operative configuration.

4. The medical system (20, 120) of claim 3, wherein the plurality of flaps (46, 146) form the fluidic seal with the tissue (14).

5. The medical system (20, 120) of claim 2, wherein the tissue (14) extends between the flaps (46, 146) and forms a fluidic seal with the medical instrument (22, 122) when the medical instrument (22, 122) is extended through the distal end (32, 132) of the overtube (24, 124).

6. The medical system (20, 120) of claim 2, wherein a portion of the overtube (24, 124) that is proximal to the plurality of flaps (46, 146) forms the fluidic seal with the tissue (14).

7. The medical system (20, 120) of claim 1, wherein the longitudinal length of the flaps (46, 146) is less than or about equal to a thickness of the tissue (14) defining the bodily opening (12).

8. The medical system (20, 120) of claim 1, wherein the medical instrument (22, 122) is an endoscope, and wherein a diameter of the overtube lumen (36) is reduced along the tapered portion (38) of the distal end (32, 132), the reduced diameter of the overtube lumen (36) being smaller than an outer diameter of the endoscope.

9. The medical system (20, 120) of claim 8, wherein a proximal end (50, 150) of each of the plurality of slits (44, 144) is located along the tapered portion (38) of the distal end (32, 132).

10. The medical system (20, 120) of claim 1, wherein the distal tip (40, 140) has a convex curvature.

11. The medical system (20, 120) of any preceding claim, wherein the plurality of slits (44, 144) have a longitudinal length that is about equal to or less than a thickness of the tissue (14).

12. The medical system (20, 120) of claim 1, wherein each of the plurality of flaps (46, 146) include side edges (48), and wherein the side edges (48) of adjacent flaps (46, 146) abut each other in the introduction configuration.

13. The medical system (20, 120) of claim 1, wherein each of the plurality of flaps (46, 146) include side edges (48), and wherein the side edges (48) of adjacent flaps (46, 146) overlap each other in the introduction configuration.

## Patentansprüche

1. Medizinisches System (20, 120) für den Zugang zu einer Körperöffnung (12), die durch Gewebe (14) definiert ist, wobei das medizinische System (20, 120) umfasst:
ein medizinisches Instrument (22, 122); und
einen Überschlauch (24, 124), der einen Überschlauchhohlraum (36) definiert, dessen Größe geeignet ist, das medizinische Instrument (22, 122) aufzunehmen, wobei der Überschlauch (24, 124) und der Überschlauchhohlraum (36) eine Längsachse (16) definieren, der Überschlauch (24, 124) ein distales Ende (32, 132) mit einem verjüngten Abschnitt (38) und einer Mehrzahl von sich längs erstreckenden Schlitzen (44, 144) aufweist, die eine Mehrzahl von Klappen (46, 146) definieren, wobei der Überschlauch (24, 124) einen Durchmesser aufweist, der entlang des verjüngten Abschnitts (38) des distalen Endes (32, 132) verringert ist,
wobei der verjüngte Abschnitt (38) des Überschlauchs (24, 124) relativ zu dem medizinischen Instrument (22, 122) eine derartige Größe aufweist, dass das distale Verschieben des medizinischen Geräts (22, 122) durch das distale Ende (32, 132) des Überschlauchs (24, 124) die Mehrzahl von Klappen (46, 146) dazu zwingt, sich radial zwischen einer Einführkonfiguration und einer Betriebskonfiguration zu bewegen,
wobei die Mehrzahl von Klappen (46, 146) an einer distalen Spitze (40, 140) des Überschlauchs (24, 124) ineinander eingreifen, um den Überschlauchhohlraum (36) in der Einführkonfiguration im Wesentlichen zu verschließen, wobei die Mehrzahl von Klappen (46, 146) radial nach außen in die Betriebskonfiguration bewegt wird,
**dadurch gekennzeichnet, dass**
die distale Spitze (40, 140) entlang einer kontinuierlichen Krümmung radial nach außen aufgeweitet ist, um eine Lippe (42, 142) zu definieren.

2. Medizinisches System (20, 120) nach Anspruch 1, wobei das distale Ende (32, 132) des Überschlauchs (24, 124) mit dem Gewebe (14) eine Fluid-Dichtung bildet.

3. Medizinisches System (20, 120) nach Anspruch 2, wobei jede der Mehrzahl von Klappen (46, 146) Seitenränder (48) aufweist, und wobei die Seitenränder (48) von benachbarten Klappen (46, 146) in der Betriebskonfiguration aneinander angrenzen.

4. Medizinisches System (20, 120) nach Anspruch 3, wobei die Mehrzahl von Klappen (46, 146) mit dem Gewebe (14) eine Fluid-Dichtung bilden.

5. Medizinisches System (20, 120) nach Anspruch 2, wobei sich das Gewebe (14) zwischen den Klappen (46, 146) erstreckt und eine Fluid-Dichtung mit dem medizinischen Instrument (22, 122) bildet, wenn das medizinische Instrument (22, 122) durch das distale Ende (32, 132) des Überschlauchs (24, 124) ausgefahren ist.

6. Medizinisches System (20, 120) nach Anspruch 2, wobei ein Abschnitt des Überschlauchs (24, 124), der sich proximal der Mehrzahl von Klappen (46, 146) befindet, eine Fluid-Dichtung mit dem Gewebe (14) bildet.

7. Medizinisches System (20, 120) nach Anspruch 1, wobei die Länge der Klappen (46, 146) in Längsrichtung geringer oder in etwa gleich einer Dicke des Gewebes (14) ist, das die Körperöffnung (12) definiert.

8. Medizinisches System (20, 120) nach Anspruch 1, wobei das medizinische Instrument (22, 122) ein Endoskop ist und wobei ein Durchmesser des Überschlauchhohlraums (36) entlang des verjüngten Abschnitts (38) des distalen Endes (32, 132) verringert ist, wobei der verringerte Durchmesser des Überschlauchhohlraums (36) kleiner ist als ein Außendurchmesser des Endoskops.

9. Medizinisches System (20, 120) nach Anspruch 8, wobei ein proximales Ende (50, 150) von jedem der Mehrzahl von Schlitzen (44, 144) entlang des verjüngten Abschnitts (38) des distalen Endes (32, 132) angeordnet ist.

10. Medizinisches System (20, 120) nach Anspruch 1, wobei die distale Spitze (40, 140) eine konvexe Krümmung aufweist.

11. Medizinisches System (20, 120) nach einem der vorangehenden Ansprüche, wobei die Mehrzahl von Schlitzen (44, 144) eine Länge in Längsrichtung aufweist, die in etwa gleich der oder geringer als eine Dicke des Gewebes (14) ist.

12. Medizinisches System (20, 120) nach Anspruch 1, wobei jede der Mehrzahl von Klappen (46, 146) Seitenränder (48) aufweist und wobei die Seitenränder (48) von benachbarten Klappen (46, 146) in der Einführkonfiguration aneinander angrenzen.

13. Medizinisches System (20, 120) nach Anspruch 1, wobei jede der Mehrzahl von Klappen (46, 146) Seitenränder (48) aufweist, und wobei sich die Seitenränder (48) von benachbarten Klappen (46, 146) in der Einführkonfiguration gegenseitig überlappen.

## Revendications

1. Système médical (20, 120) pour accéder à une ouverture corporelle (12) délimitée par un tissu (14), le système médical (20, 120), comprenant :
un instrument médical (22, 122) ; et
un overtube (24, 124) délimitant une lumière (36) d'overtube dimensionnée pour recevoir l'instrument médical (22, 122), l'overtube (24, 124) et la lumière (36) d'overtube définissant un axe longitudinal (16), l'overtube (24, 124) comprenant une extrémité distale (32, 132) comportant une partie conique (38) et une pluralité de fentes (44, 144) s'étendant longitudinalement et délimitant une pluralité de rabats (46, 146), l'overtube (24, 124) ayant un diamètre qui se réduit le long de la partie conique (38) de l'extrémité distale (32, 132),
la partie conique (38) de l'overtube (24, 124) étant dimensionnée par rapport à l'instrument médical (22, 122) de telle sorte que la translation distale de l'instrument médical (22, 122) à travers l'extrémité distale (32, 132) de l'overtube (24, 124) force la pluralité de rabats (46, 146) à se déplacer radialement entre une configuration d'introduction et une configuration fonctionnelle,
la pluralité de rabats (46, 146) s'appliquant l'un sur l'autre au niveau d'une pointe distale (40, 140) de l'overtube (24, 124) pour fermer pratiquement la lumière (36) d'overtube dans la configuration d'introduction, la pluralité de rabats (46, 146) étant déplacés radialement vers l'extérieur dans la configuration fonctionnelle,
**caractérisé en ce que** la pointe distale (40, 140) est évasée radialement vers l'extérieur suivant une courbure continue pour définir une lèvre (42, 142).

2. Système médical (20, 120) selon la revendication 1, dans lequel l'extrémité distale (32, 132) de l'overtube (24, 124) forme un joint d'étanchéité fluidique avec le tissu (14).

3. Système médical (20, 120) selon la revendication 2, dans lequel chaque rabat de la pluralité de rabats (46, 146) comprend des bords latéraux (48) et dans lequel les bords latéraux (48) de rabats (46, 146) adjacents viennent buter l'un contre l'autre dans la configuration fonctionnelle.

4. Système médical (20, 120) selon la revendication 3, dans lequel la pluralité de rabats (46, 146) constituent le joint d'étanchéité fluidique avec le tissu (14).

5. Système médical (20, 120) selon la revendication 2, dans lequel le tissu (14) s'étend entre les rabats (46, 146) et forme un joint d'étanchéité fluidique avec l'instrument médical (22, 122) quand on pousse l'instrument médical (22, 122) à travers l'extrémité distale (32, 132) de l'overtube (24, 124).

6. Système médical (20, 120) selon la revendication 2, dans lequel la partie de l'overtube (24, 124) qui est proximale à la pluralité de rabats (46, 146) constitue le joint d'étanchéité fluidique avec le tissu (14).

7. Système médical (20, 120) selon la revendication 1, dans lequel la longueur longitudinale des rabats (46, 146) est inférieure ou à peu près égale à l'épaisseur du tissu (14) délimitant l'ouverture corporelle (12).

8. Système médical (20, 120) selon la revendication 1, dans lequel l'instrument médical (22, 122) est un endoscope et dans lequel le diamètre de la lumière (36) d'overtube se réduit le long de la partie conique (38) de l'extrémité distale (32, 132), le diamètre réduit de la lumière (36) d'overtube étant inférieur au diamètre extérieur de l'endoscope.

9. Système médical (20, 120) selon la revendication 8, dans lequel l'extrémité proximale (50, 150) de la pluralité de fentes (44, 144) est située le long de la partie conique (38) de l'extrémité distale (32, 132).

10. Système médical (20, 120) selon la revendication 1, dans lequel la pointe distale (40, 140) a une courbure convexe.

11. Système médical (20, 120) selon l'une quelconque des revendications précédentes, dans lequel la pluralité de fentes (44, 144) ont une longueur longitudinale qui est inférieure ou à peu près égale à l'épaisseur du tissu (14).

12. Système médical (20, 120) selon la revendication 1, dans lequel chaque rabat de la pluralité de rabats (46, 146) comprend des bords latéraux (48) et dans lequel les bords latéraux (48) de rabats (46, 146) adjacents viennent buter l'un contre l'autre dans la configuration d'introduction.

13. Système médical (20, 120) selon la revendication 1, dans lequel chaque rabat de la pluralité de rabats (46, 146) comprend des bords latéraux (48) et dans lequel les bords latéraux (48) de rabats (46, 146) adjacents se chevauchent l'un l'autre dans la configuration d'introduction.
